(19) Europäisches Patentamt
European Patent Office
Office européen des brevets

(11) EP 2 046 316 B1

(12) EUROPEAN PATENT SPECIFICATION

(45) Date of publication and mention of the grant of the patent:
**29.09.2010 Bulletin 2010/39**

(21) Application number: **06792670.9**

(22) Date of filing: **03.08.2006**

(51) Int Cl.:
***A61K 31/16*** $^{(2006.01)}$ ***A61P 31/12*** $^{(2006.01)}$

(86) International application number:
**PCT/EP2006/065035**

(87) International publication number:
**WO 2008/014824 (07.02.2008 Gazette 2008/06)**

# (54) ANTIVIRAL USE OF CATIONIC SURFACTANT

ANTIVIRALE VERWENDUNG VON KATIONISCHEM TENSID

UTILISATION ANTIVIRALE DE TENSIOACTIF CATIONIQUE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**15.04.2009 Bulletin 2009/16**

(73) Proprietor: **Laboratorios Miret, S.A.**
**08228 Les Fonts de Terrassa, Barcelona (ES)**

(72) Inventors:
- **ROCABAYERA BONVILA, Xavier**
  **08184 Palau-Solita / Barcelona (ES)**
- **FIGUERAS ROCA, Sergi**
  **08005 Barcelona (ES)**
- **SEGRET PONS, Roger**
  **08023 Barcelona (ES)**

(74) Representative: **Gille Hrabal Struck Neidlein Prop Roos**
**Patentanwälte**
**Brucknerstrasse 20**
**40593 Düsseldorf (DE)**

(56) References cited:
**WO-A-03/043593** **WO-A-2006/125121**

- **RUCKMAN ET AL: "Toxicological and metabolic investigations of the safety of N-lauroyl L arginine ethyl ester monohydrochloride" FOOD AND CHEMICAL TOXICOLOGY, vol. 42, 2004, - 2004 pages 245-259, XP002420948**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 046 316 B1

## Description

The present application relates to a novel use of cationic surfactants.

Cationic surfactants are well-known in the art for a variety of different applications.

Cationic surfactants derived from lauric acid and arginine, in particular the ethyl ester of the lauramide of the arginine monohydrochloride, hereafter named LAE, may be used for the protection against the growth of the microorganisms. The chemical structure of LAE is described in the following formula (1):

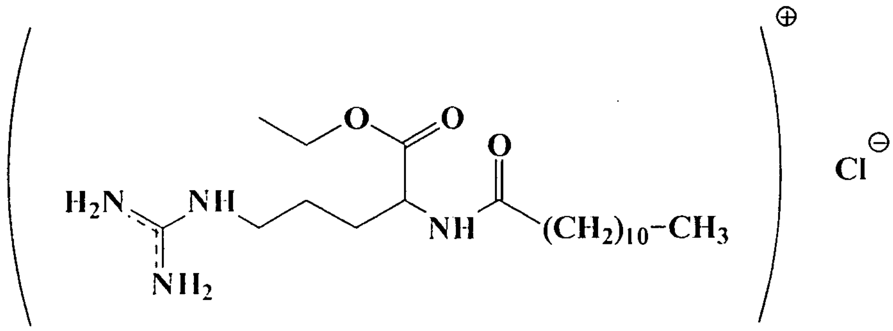

The preparation of this product has been described in Spanish patent application ES-A-512643.

The metabolism of the above cationic surfactant of formula (1) in rats has been studied, these studies have shown a fast absorption and metabolisation into naturally-occurring amino acids and the fatty acid lauric acid, which are eventually excreted as carbon dioxide and urea. Toxicological studies have demonstrated, that LAE is completely harmless to animals and humans.

Therefore, LAE and related compounds are particularly suitable to be used in the preservation of all perishable food products. LAE and related compounds are equally suitable for use in cosmetic products.

This compound is remarkable for its inhibitory action over the proliferation of different microorganisms, such as bacteria, fungi and yeasts. The minimum inhibitory concentrations of LAE are shown in the following table 1.

**Table 1**

| Kind | Microorganism | M.I.C. (ppm) |
|---|---|---|
| Gram + Bacteria | *Arthrobacter oxydans* ATCC 8010 | 64 |
| | *Bacillus cereus var mycoide* ATCC 11778 | 32 |
| | *Bacillus subtilis* ATCC 6633 | 16 |
| | *Clostridium perfringens* ATCC 77454 | 16 |
| | *Listeria monocytogenes* ATCC 7644 | 10 |
| | *Staphylococcus aureus* ATCC 6538 | 32 |
| | *Micrococcus luteus* ATCC 9631 | 128 |
| | *Lactobacillus delbrueckii ssp lactis* CECT 372 | 16 |
| | *Leuconostoc mesenteroides CETC 912* | 32 |
| Gram - Bacteria | *Alcaligenes faecalis* ATCC 8750 | 64 |
| | *Bordetella bronchiseptica* ATCC 4617 | 128 |
| | *Citrobacter freundii* ATCC 22636 | 64 |
| | *Enterobacter aerogenes* CECT 689 | 32 |
| | *Escherichia coli* ATCC 8739 | 32 |
| | *Escherichia coli* 0157H7 | 20 |
| | *Klebsiella pneumoniae var pneumoniae* CECT 178 | 32 |
| | *Proteus mirabilis* CECT 170 | 32 |
| | *Pseudomonas aeruginosa* ATCC 9027 | 64 |
| | *Salmonella typhimurium* ATCC16028 | 32 |
| | *Serratia marcenses* CECT 274 | 32 |
| | *Mycobacterium phlei* ATCC 41423 | 2 |

(continued)

| Kind | Microorganism | M.I.C. (ppm) |
|---|---|---|
| **Fungi** | *Aspergillus niger* ATCC14604 | 32 |
| | *Aureobasidium pullulans* ATCC 9348 | 16 |
| | *Gliocadium virens* ATCC 4645 | 32 |
| | *Chaetonium globosum* ATCC 6205 | 16 |
| | *Penicillium chrysogenum* CECT 2802 | 128 |
| | *Penicillium funiculosum* CECT 2914 | 16 |
| **Yeast** | *Candida albicans* ATCC 10231 | 16 |
| | *Rhodotorula rubra* CECT 1158 | 16 |
| | *Saccharomyces cerevisiae* ATCC 9763 | 32 |

The use of the invention relates to cationic surfactants derived from the condensation of fatty acids and esterified dibasic amino acids, according to the following formula (2):

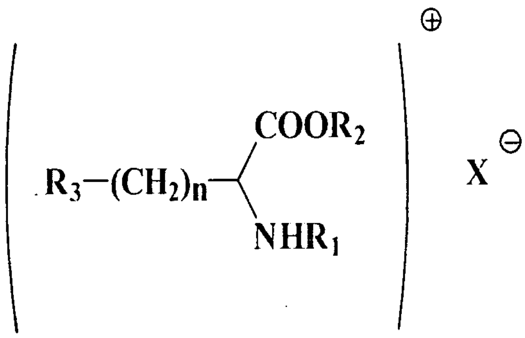

where:

$X^-$ is $Br^-$, $Cl^-$, $HSO_4^-$ , a counter ion derived from an organic or inorganic acid, or an anion on the basis of a phenolic compound;

$R_1$: is a linear alkyl chain from a saturated fatty acid or hydroxyacid from 8 to 14 atoms of carbon bonded to the α-amino acid group through an amidic bond;

$R_2$: is a linear or branched alkyl chain from 1 to 18 carbon atoms or an aromatic group;

$R_3$: is

**$-NH_3$**

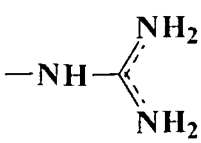

or

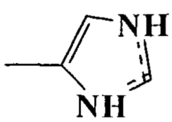

and n can be from 0 to 4.

The organic acids which may be the source of the counter ion X-can be citric acid, lactic acid, acetic acid, fumaric acid, maleic acid, gluconic acid, propionic acid, sorbic acid, benzoic acid, carbonic acid, glutamic acid or other amino acids, lauric acid and fatty acids such as oleic acid and linoleic acid, whereas the inorganic acids can be phosphoric acid, nitric acid and thiocyanic acid.

The phenolic compound which may be the basis of the anion X- is for instance butylated hydroxyanisole (BHA) and the related butylated hydroxytoluene, tertiary butyl hydroquinone and parabens such as methylparaben, ethylparaben, propylparaben and butylparaben.

The most preferred compound of the above class of compounds is LAE.

It is preferred to dissolve the compound directly before use in one of the following preferred solvents of food grade: water, ethanol, propylene glycol, isopropyl alcohol, other glycols, mixtures of glycols and mixtures of glycols and water. If the treatment shall be performed at a specific pH value the use of a corresponding buffer solution may be recommendable. On the other hand the compound can be easily used as a solid. Surfaces which shall be protected by solid preparations are for instance the surfaces of food products or cosmetics.

For the cationic surfactants of the above formula (2) the antibacterial activity and the biological activity against other microorganisms such as fungi and yeasts is well documented. An activity of the cationic preservatives against viruses has not been described.

Safe and effective products for the treatment of viral infections are urgently and constantly needed. World-wide outbreaks of virus infections illustrate the complexity of effective treatments. Recently the outbreak of avian flu led to an urgent search for the optimum tools to limit the spreading of the disease.

Avian influenza, nowadays also known as avian flu, is a bird disease caused by influenza A viruses, essentially of haemagglutinin subtypes H5 or H7, which belong to the family *Orthomyxoviridae.* Within the same group of viruses the virus responsible for the respiratory disease called influenza or flu is found.

Parainfluenza viruses, included in subfamily *Paramyxovirinae,* with the family of the *Paramyxoviridae,* include viruses affecting humans as a well as animals. Virions are 150 nm in diameter, pleomorphic, with a lipid envelope derived from the host cell membrane and with projections of 10-14 nm in length. Its genome contains 7-8 segments of linear, negative sense, single stranded RNA, resulting in a genome size of 10,000 to 14,600 nucleotides.

Furthermore, *Paramyxoviridae* and *Orthomyxoviridae* share special relationships with respect to the biological properties of the envelope glycoproteins. As seen in the following table 2, *Paramyxoviridae* and *Orthomyxoviridae* do not display major differences in their protein, lipid and carbohydrate content.

Table 2

Protein, lipid and carbohydrate content of members of families *Paramyxoviridae* and *Orthomyxoviridae.*

| Content (%) | *Paramyxoviridae* | *Orthomyxoviridae* |
|---|---|---|
| Proteins | 70-74 | 70-77 |
| Lipids | 20-25 | 18-37 |
| Carbohydrates | 6 | 5-8 |

The term parainfluenza was originally chosen, because some of the disease symptoms are highly similar to those of influenza and both virus types display hemagglutination and neuraminidase activities.

Influenza virus and parainfluenza virus display the same sensitivity against external influences such as heat.

It is a belief in the art, that parainfluenza virus is a good experimental model for the determination of the biological activity of test compounds against influenza virus.

It is the unexpected result of the investigations made by the inventors, that the cationic surfactants of above formula (2) displayed a surprising, remarkably strong antiviral activity. Until now this class of compounds was known for its antimicrobial action and an antivirus action could not be expected.

The antiviral activity could be observed against viruses of *Vaccinia, Herpes simplex* and *bovine parainfluenzae virus* types. Particularly strong was the action against viruses of the *Herpes* and *bovine parainfluenzae virus* types, in which types a short contact time of 5 minutes was sufficient for achieving maximum effects. This may be considered as the particularly surprising aspect of the present invention, of achieving the effect against the viruses after such a short time.

There is a similarity between the virus types which have turned out to be most sensitive to the antiviral action of the cationic surfactants such as the *Vaccinia, Herpes simplex* and *bovine parainfluenzae virus* types and a virus such the adenovirus which is the agent which is responsible for AIDS. The cationic surfactants which have been investigated by the present inventors are found to be effective against *human immunodeficiency virus I* as well.

The present invention relates to the use of the cationic surfactants of formula (2) as treating any kind of products which are affected by a virus contamination. Such products may for instance be warm dishes or warm liquids. The products to be treated may be any kind of equipment which is used in the handling of animals which are infected with

virus. In an even broader sense the products to be treated can be the facilities where animals are kept, or parts of the natural environment such as the ground surface or water reservoirs.

The present invention furthermore relates to compositions for administration of the cationic surfactants of formula (2) to animals or human beings directly, for prophylactic or therapeutic treatment of virus diseases.

The cationic surfactants of formula (2) may be applied as a solution. This is the easy and suitable manner of treating the surface of the ground, cars and people. For other applications it may be more suitable to apply the cationic surfactant as a solid, which may be equally effective.

The treatment of products in order to avoid any kind of virus infection might involve the presence of a concentration of the cationic surfactants of the formula (2), more in particular according to the preferred embodiment of LAE, of around 2 to 20,000 ppm of the product to be protected, preferably a concentration of 100 to 10,000 ppm, and more preferably 200 to 2,000 ppm. This is a similar concentration as has been described for achieving the microbiocidal action. Products to be treated with the above-indicated range of concentrations of the cationic surfactants are for instance food products or cosmetics.

The treatment of surfaces which are infected with viruses, such as the surface of food preparations, the surface of cosmetics, ground surface, the surface of any kind of vehicles, and the surface of any equipment used in the handling of animals infected with virus, requires the presence of the cationic surfactants of the formula (2), more in particular according to the preferred embodiment of LAE, at a concentration of a level which is sufficient to achieve the wanted antiviral action at such surfaces. Such level of concentration would be expected in the range of 2 to 20,000 ppm, more preferred 100 to 10,000 ppm and even more preferred 200 to 2,000 ppm, containing the cationic surfactant of claims, according to the preferred embodiment containing LAE. These concentrations are given in terms of the concentration of a solution containing the cationic surfactant which is applied to the surfaces to be treated. If surfaces are treated with a solid preparation of the cationic surfactant of formula (2), the amount which is applied shall be such that the amount of the cationic surfactant of formula (2) shall be in the range of 0.01 to 100 $mg/dm^2$, preferably an amount of 0.5 to 50 $mg/dm^2$, and more preferably an amount of 1 to 10 $mg/dm^2$.

The treatment of liquid preparations such as drinking fluids or natural sources of water such as lakes or ponds requires the presence of the cationic surfactants of the formula (2), more in particular according to the preferred embodiment of LAE, at a concentration of a level which is sufficient to achieve the wanted antiviral action in the drinking fluid or water. Such level of concentration would be expected in the range of 0.2 to 20,000 ppm, more preferred 2 to 15,000 ppm, even more preferred 100 to 10,000 ppm and most preferred 200 to 2,000 ppm, containing the cationic surfactant of formula (2), according to the preferred embodiment containing LAE. These concentrations are provided in terms of the concentration of the cationic surfactant in the liquid or the water to be treated.

The treatment of animals or humans implies the administration of the cationic surfactants in a manner which is suitable for absorption of compounds used according to the invention. The compounds can be administered orally, parenterally (including intraperitoneal, subcutaneous and intramuscular injections) or externally (topically, such as rectal, transdermal, by instillation and transnasal). The preparation to be administered may have the form of a conventional pharmaceutical preparation such as capsules, microcapsules, tablets, enteric coated agents, granules, powder, pills, ointments, suppositories, suspensions, syrups, emulsions, liquids, sprays, inhalants, eye drops and nose drops.

The above-mentioned pharmaceutical preparations can be produced according to conventional methods using various organic or inorganic carriers conventionally used for the pharmaceutical formulation of preparations, such as excipients (such as sucrose, starch, mannit, sorbite, lactose, glucose, cellulose, talc, calcium phosphate, calcium carbonate), binders (such as cellulose, methyl cellulose, hydroxymethyl cellulose, polypropylpyrrolidone, gelatin, Arabic gum, polyethylene glycol, sucrose, starch), disintegrants (such as starch, carboxymethyl cellulose, hydroxypropyl starch, sodium hydrogen carbonate, calcium phosphate, calcium citrate), lubricants (such as magnesium stearate, aerosyl, talc, sodium lauryl sulphate), corrigents (such as citric acid, menthol, glycine, orange powder), preservatives (sodium benzoate, sodium bisulfite, methylparaben, propylparaben), stabilizers (such as citric acid, sodium citrate, acetic acid), suspending agents (methyl cellulose, polyvinylpyrrolidone, aluminium stearate), dispersing agents (such as hydroxypropylmethyl cellulose), diluents (such as water), base waxes (such as cacao butter, white petrolatum, polyethylene glycol) and other suitable ones.

The dose of the cationic surfactant according to the use of the present invention shall be determined by the dose required for achieving the wanted prophylactic or therapeutic effect. A usual dose shall be 0,1 mg/kg to 10 mg/kg for oral or parenteral administration. A usual dose in humans may be a unit dose of 0,1 to 1000 mg per individual, more preferable 0,5 to 500 mg per individual. This dose may be administered 1 to 4 times per day, depending on the severity of the symptoms. A usual dose in animals may be 0,1 to 100 mg per dose, preferred 0,5 to 50 mg per dose.

The effect of the use of the invention is illustrated through the following examples.

In the following experiments the reduction factor has been calculated.

This reduction factor is calculated according to the following formula

$$10^{R'} = \frac{v' \cdot 10^{a'}}{v'' \cdot 10^{a''}}$$

In this formula:

R' presents the reduction factor,
v' represents the volume of the sample of the test compound,
a' is the virus concentration in the added sample ($^{10}$log value),
v" is the volume of the final sample,
a" is the virus concentration after treatment ($^{10}$log value).

In the following experiments the clearance factor has been calculated. The clearance factor is calculated according to the following formula:

$$10^{CL} = \frac{10^{q'}}{10^{q''}},$$

In this formula:

CL is the elimination factor (clearance factor),
q' is the initial concentration of virus ($^{10}$log value),
q" is the final concentration of virus ($^{10}$log value).

Example 1.

**The activity of LAE was investigated against the *virus bovine parainfluenzae 3 virus.***

Medium for cell cultivation

Eagle minimum essential medium (EMEM) was obtained from Earle (ICN Flow, Rf. 11-100-24), was supplemented with 10 % of fetal bovine serum (Biowhittaker, Ref. D14-810F),
2 nM of glutamine (Biowhittaker, Ref. 17-605E),
non-essental amino acids (Biowhittaker, Ref. 13-114E),
100 units of penicilline,
100 μg of streptomycine/ml and
1mM of sodium pyruvate.
LAE (CAS 60372-77-2) lot no. 10234 with a LAE content of 88,7%.
The investigated strain of the *parainfluenza bovine type* 3 was SF-4 (ATCC VR-281).

Experimental conditions:

LAE was investigated at a concentration of 200 ppm.
All experiments were performed at room temperature.
8 ml of deionized water was mixed with 1 ml of the virus suspension. 1 ml of LAE solution (containing 2000 ppm of LAE) was added, leading to a final LAE concentration of 200 ppm.
After 5 and 60 minutes samples for determination were taken. The design of the experiment allowed for conclusions of the effectivity of LAE after contact times of 5 and 60 minutes.
The results are displayed in the following table 3.
Table 3.

| Virus concentration | |
|---|---|
| Before addition of LAE | 8,09 ± 0,33 |
| 60 min after addition of LAE | ≤ 1,63 |
| Reduction factor | ≥ 6,46 ± 0,32 |
| Clearance factor | ≥ 6,29 ± 0,32 |

After treatment with LAE for 60 minutes no remaining virus organisms were detected. Similar results were achieved after 5 minutes.

The number of virus particles in the experiment were determined by the quantitative assay TCID50 (infectious dose of cultured tissue).

Example 2.

**Activity of LAE against *Herpes virus type 1.***

The virus which was investigated was the type *Simplex virus* of the subfamily *Alphaherpesvirinae* in the family *Herpesviridae.*

The conditions for the determination are similarly chosen according to the ones of example 1.

LAE was investigated at a concentration of 200 ppm.

The results are displayed in the following table 4.

Table 4.

| Virus concentration | |
|---|---|
| Before addition of LAE | 7,94 ± 0,37 |
| 60 min after addition of LAE | ≤ 1,70 |
| Reduction factor | ≥ 6,18 ± 0,36 |
| Clearance factor | ≥ 6,24 ± 0,37 |

After treatment with LAE for 60 minutes no remaining virus organisms were detected. Similar results were achieved after 5 minutes.

Example 3.

**Activity of LAE against *Vaccinia virus.***

The virus which was investigated was the type *Orthopoxvirus* from the family *Poxviridae.*

The family of the *Poxviridae* covers complex viruses having a complex structure with a nucleus containing viral DNA (double chain with 170-250 kbp).

LAE was investigated at a concentration of 200 ppm.

The results are displayed in the following table 5.

Table 5.

| Virus concentration | |
|---|---|
| Before addition of LAE | 5,94 ± 0,34 |
| 60 min after addition of LAE | 2,40 |
| Reduction factor | 3,92 ± 0,36 |
| Clearance factor | 3,54 ± 0,34 |

After treatment with LAE for 60 minutes a substantial decrease of the number of living *Vaccinia virus* organisms

was detected. A great reduction of the number of living microorganisms was also observed after treatment for 5 minutes.

**Claims**

1. A cationic surfactant derived from the condensation of fatty acids and esterified dibasic amino acids, according to the following formula (2):

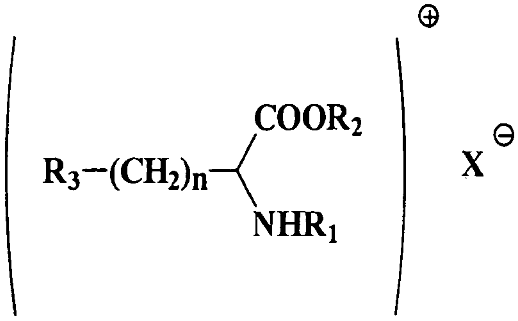

where:

$X^-$ is $Br^-$, $Cl^-$ , or $HSO_4^-$, a counter ion derived from an organic or inorganic acids, or an anion on the basis of a phenolic compound;
$R_1$: is a linear alkyl chain from a saturated fatty acid or hydroxyacid from 8 to 14 atoms of carbon bonded to the α- amino acid group through an amidic bond,
$R_2$: is a linear or branched alkyl chain from 1 to 18 carbon atoms or
an aromatic group,
$R_3$: is
**$-NH_3$**

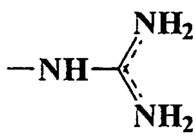

or

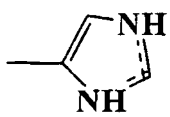

and n can be from 0 to 4,
for the use for the treatment of virus infections.

2. The cationic surfactant of claim 1, for the manufacture of a medicament for the treatment of virus infections in animals or human beings.

3. The cationic surfactant of claim 2, which is administered orally, parenterally (including intraperitoneal, subcutaneous and intramuscular injections) or externally (topically, such as rectal, transdermal, by instillation or transnasal).

4. The cationic surfactant of any of the claims 1 to 3, which is the ethyl ester of the lauramide of the arginine monohydrochloride (LAE).

**5.** The cationic surfactant of any of claims 2 to 4, for the use for the treatment of a disease caused by an influenza virus.

**6.** The cationic surfactant of claim 5, for the use for the treatment of a disease caused by an influenza virus which is causing avian irifluenza.

**7.** The cationic surfactant of any of claims 2 to 6 for the use for prophylactic application to animals or human beings.

**8.** The cationic surfactant of any of claims 1 to 7 for the use for the treatment of virus infections in which the virus is a *Vaccinia, Herpes simplex* and *bovine parainfluenzae virus* type virus.

**9.** Use of a cationic surfactant derived from the condensation of fatty acids and esterified dibasic amino acids, according to the following formula (2):

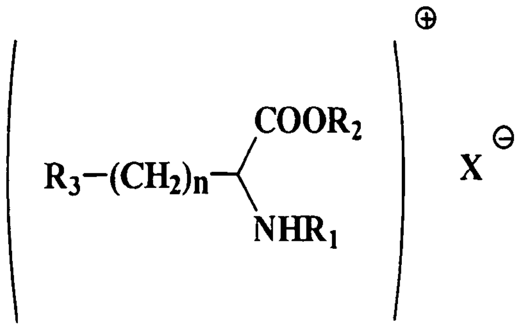

where:

$X^-$ is $Br^-$, $Cl^-$ , or $HSO_4^-$, a counter ion derived from an organic or inorganic acids, or an anion on the basis of a phenolic compound;
$R_1$: is a linear alkyl chain from a saturated fatty acid or hydroxyacid from 8 to 14 atoms of carbon bonded to the α- amino acid group through an amidic bond,
$R_2$: is a linear or branched alkyl chain from 1 to 18 carbon atoms or
an aromatic group,
$R_3$: is
**$-NH_3$**

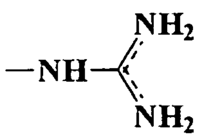

or

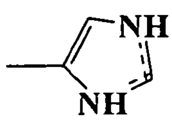

and n can be from 0 to 4,
for the treatment of surfaces and preparations which are infected with viruses, such as the surface of food preparations, the surface of cosmetics, ground surface, the surface of any kind of vehicles, and the surface of any equipment used in the handling of animals infected with virus.

**10.** The use of claim 9 for the treatment of liquid preparations such as drinking fluids or natural sources of water.

**11.** The use of claim 9 for the treatment of food preparations.

**12.** The use of claim 9 for the treatment of cosmetic preparations.

**13.** The use of any of the claims 9 to 12, in which the virus is a *Vaccinia, Herpes simplex* and *bovine parainfluenzae virus* type virus.

**Patentansprüche**

**1.** Kationisches oberflächenaktives Mittel, erhalten aus der Kondensation von Fettsäuren und veresterten dibasischen Aminosäuren entsprechend der folgenden Formel (2)

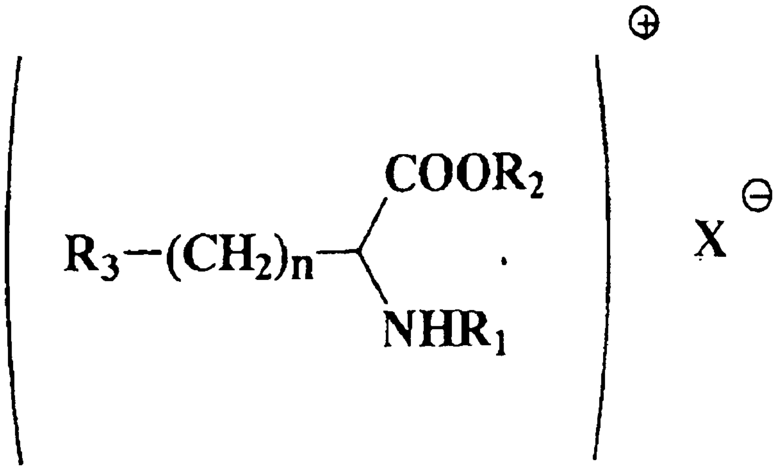

wobei:

$X^-$ $Br^-$, $Cl^-$ oder $HSO_4^-$, ein von einer organischen oder anorganischen Säure abgeleitetes Gegenion, oder ein Anion auf der Basis einer phenolischen Verbindung ist;
$R_1$ eine lineare Alkylkette einer gesättigten Fettsäure oder Hydroxysäure mit 8 bis 14 Kohlenstoffatomen ist, die an die α-Aminogruppe durch eine Amidbindung gebunden ist,
$R_2$ eine lineare oder verzweigte Alkylkette mit 1 bis 18 Kohlenstoffatomen oder eine aromatische Gruppe ist,
$R_3$
**$-NH_3$**

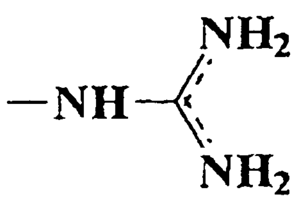

oder

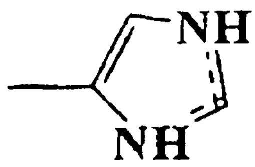

ist
und n 0 bis 4 sein kann,
zur Verwendung für die Behandlung von Virusinfektionen.

**2.** Kationisches oberflächenaktives Mittel nach Anspruch 1 zur Herstellung eines Medikaments zur Behandlung von Virusinfektionen bei Tieren oder Menschen.

**3.** Kationisches oberflächenaktives Mittel nach Anspruch 2, welches oral, parenteral (einschließlich intraperitonale, subkutane und intramuskuläre Injektionen) oder extern (topisch, wie rektal, transdermal, durch Instillation oder transnasal) verabreicht wird.

**4.** Kationisches oberflächenaktives Mittel nach einem der Ansprüche 1 bis 3, welches der Ethylester des Lauramids von Argininmonohydrochlorid ist (LAE).

**5.** Kationisches oberflächenaktives Mittel nach einem der Ansprüche 2 bis 4 zur Verwendung für die Behandlung einer durch einen Influenza-Virus verursachten Erkrankung.

**6.** Kationisches oberflächenaktives Mittel nach Anspruch 5 zur Verwendung für die Behandlung einer Erkrankung, die durch ein Influenza-Virus verursacht wird, welches Vogelgrippe verursacht.

**7.** Kationisches oberflächenaktives Mittel nach einem der Ansprüche 2 bis 6 zur Verwendung für die prophylaktische Anwendung bei Tieren oder Menschen.

**8.** Kationisches oberflächenaktives Mittel nach einem der Ansprüche 1 bis 7 zur Verwendung für die Behandlung von Virusinfektionen, bei denen der Virus ein *Vaccinia-, Herpes simplex-* und *Rinderparainfluenzavirus-Typ-Virus* ist.

**9.** Verwendung eines kationischen oberflächenaktiven Mittels, das aus der Kondensation von Fettsäuren und veresterten dibasischen Aminosäuren entsprechend der folgenden Formel (2) abgeleitet ist:

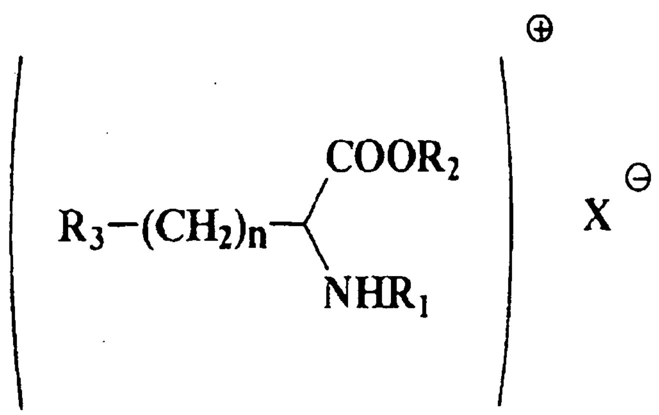

wobei
$X^-$ $Br^-$, $Cl^-$ oder $HSO_4^-$, ein von einer organischen oder anorganischen Säure abgeleitetes Gegenion, oder ein Anion auf der Basis einer phenolischen Verbindung ist;
$R_1$ eine lineare Alkylkette einer gesättigten Fettsäure oder Hydroxysäure mit 8 bis 14 Kohlenstoffatomen ist, die an

die α-Aminogruppe durch eine Amidbindung gebunden ist,
$R_2$ eine lineare oder verzweigte Alkylkette mit 1 bis 18 Kohlenstoffatomen oder eine aromatische Gruppe ist,
$R_3$
**$-NH_3$**

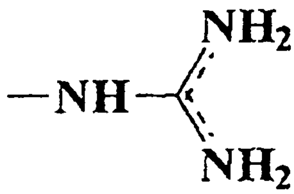

oder

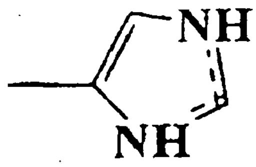

ist
und n 0 bis 4 sein kann,
zur Behandlung von Oberflächen und Zubereitungen, die mit Viren infiziert sind, wie die Oberflächen von Lebensmittelzubereitungen, die Oberfläche von Kosmetika, Bodenoberfläche, die Oberfläche jeder Art Fahrzeuge und die Oberfläche aller Vorrichtungen, die bei der Handhabung von mit Viren infizierten Tieren verwendet werden.

**10.** Verwendung nach Anspruch 9 zur Behandlung von flüssigen Zubereitungen wie Trinkflüssigkeiten oder natürlichen Wasserquellen.

**11.** Verwendung nach Anspruch 9 zur Behandlung von Lebensmittelzubereitungen.

**12.** Verwendung nach Anspruch 9 zur Behandlung von kosmetischen Präparaten.

**13.** Verwendung nach einem der Ansprüche 9 bis 12, wobei der Virus ein *Vaccinia-, Herpes simplex-* und *Rinderparainfluenza-Virus-Typ-Virus* ist.

**Revendications**

**1.** Tensioactif cationique dérivé de la condensation d'acides gras et d'acides aminés dibasiques estérifiés, répondant à la formule (2) suivante :

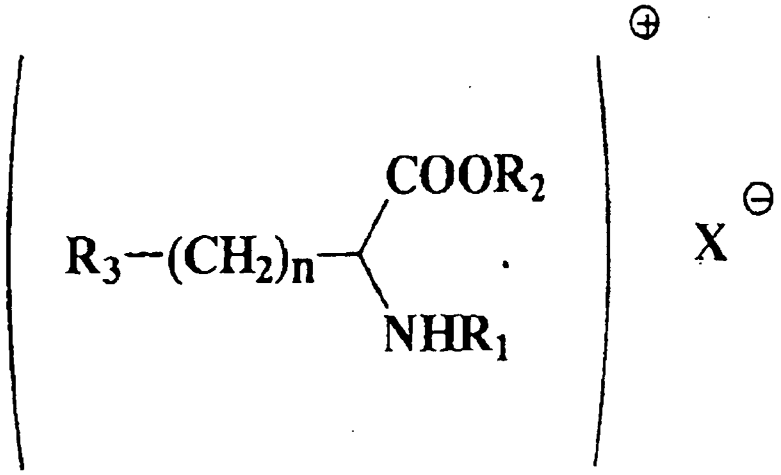

dans laquelle :

$X^-$ représente un ion $Br^-$, $Cl^-$ ou $HSO_4^-$, un contre-ion dérivé d'un acide organique ou inorganique ou un anion sur la base d'un composé phénolique ;
$R_1$ représente une chaîne alkyle linéaire d'un acide gras saturé ou d'un hydroxyacide de 8 à 14 atomes de carbone reliée au groupe acide α-aminé par une liaison amidique,
$R_2$ représente une chaîne alkyle linéaire ou ramifiée de 1 à 18 atomes de carbone ou un groupe aromatique,
$R_3$ représente
**$-NH_3$**

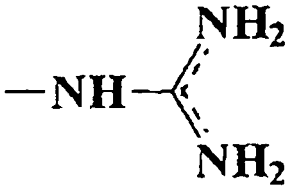

ou

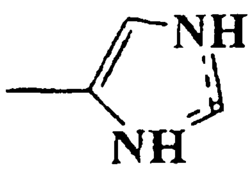

et n peut être de 0 à 4,
destiné à une utilisation dans le traitement des infections virales.

**2.** Tensioactif cationique selon la revendication 1, destiné à la fabrication d'un médicament pour le traitement des infections virales chez les animaux ou les êtres humains.

**3.** Tensioactif cationique selon la revendication 2, qui est administré par voie orale, par voie parentérale (notamment injections intra-péritonéales, sous-cutanées et intramusculaires) ou par voie externe (par voie topique, notamment

par voie rectale, transdermique, par instillation ou par voie transnasale).

4. Tensioactif cationique selon l'une quelconque des revendications 1 à 3, qui est l'ester éthylique du lauramide du monochlorhydrate d'arginine (LAE).

5. Tensioactif cationique selon l'une quelconque des revendications 2 à 4, destiné à une utilisation dans le traitement d'une maladie provoquée par un virus de la grippe.

6. Tensioactif cationique selon la revendication 5, destiné à une utilisation dans le traitement d'une maladie provoquée par un virus de la grippe qui entraîne une grippe aviaire.

7. Tensioactif cationique selon l'une quelconque des revendications 2 à 6 destiné à l'application prophylactique à des animaux ou à des êtres humains.

8. Tensioactif cationique selon l'une quelconque des revendications 1 à 7 destiné à une utilisation dans le traitement des infections virales dans lesquelles le virus est un virus de type *Vaccina, Herpes simplex* et para-influenza bovin.

9. Utilisation d'un tensioactif cationique dérivé de la condensation d'acides gras et d'acides aminés dibasiques estérifiés, répondant à la formule (2) suivante :

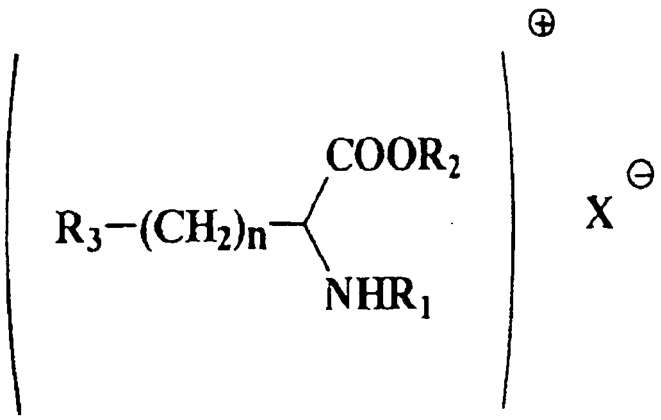

dans laquelle :

X- représente un ion $Br^-$, $Cl^-$ ou $HSO_4^-$, un contre-ion dérivé d'un acide organique ou inorganique ou un anion sur la base d'un composé phénolique ;
$R_1$ représente une chaîne alkyle linéaire d'un acide gras saturé ou d'un hydroxyacide de 8 à 14 atomes de carbone reliée au groupe acide $\alpha$-aminé par une liaison amidique,
$R_2$ représente une chaîne alkyle linéaire ou ramifiée de 1 à 18 atomes de carbone ou un groupe aromatique,
$R_3$ représente
**$-NH_3$**

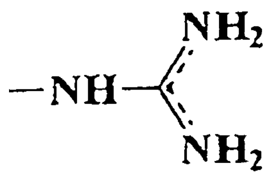

ou

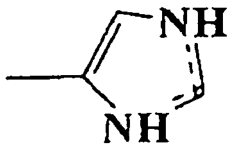

et n peut être de 0 à 4,
pour le traitement de surfaces et de préparations qui sont infectées par des virus, telles que la surface des préparations alimentaires, la surface des produits cosmétiques, la surface des sols, la surface des véhicules de tout type et la surface de tout équipement destiné à une utilisation dans la manipulation d'animaux infectés par le virus.

**10.** Utilisation selon la revendication 9 pour le traitement de préparations liquides telles que les boissons ou les sources naturelles d'eau.

**11.** Utilisation selon la revendication 9 pour le traitement des préparations alimentaires.

**12.** Utilisation selon la revendication 9 pour le traitement des préparations cosmétiques.

**13.** Utilisation selon l'une quelconque des revendications 9 à 12, dans laquelle le virus est un virus de type *Vaccinia, Herpes simplex* et para-influenza bovin.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- ES 512643 A